# EUROPEAN PATENT SPECIFICATION

(11) **EP 2 911 732 B1**
(45) Date of publication and mention of the grant of the patent: **29.11.2017**
(21) Application number: 13848277.3
(22) Date of filing: 25.10.2013
(51) Int. Cl.: A61M 25/14

(54) **APPARATUS FOR AIDING ORGAN TREATMENT**
VORRICHTUNG ZUR UNTERSTÜTZUNG EINER ORGANBEHANDLUNG
APPAREIL POUR FACILITER LE TRAITEMENT D'ORGANE

(30) Priority: 28.10.2012 US 201261719435 P
(43) Date of publication of application: 02.09.2015
(73) Proprietor: Edge Medical, LLC, Tallahassee, FL 32309 (US)
(72) Inventor: CARPENTER, Colin M., San Carlos, CA 94070 (US); LAMOUREUX, Gary A., Woodbury, CT 06798 (US); DIAZ, Juan-Carlos, Pembroke Pines, FL 33029 (US); JAHRMARKT, Scott L., Miami Beach, FL 33141 (US)
(74) Representative: Maschio, Antonio
(86) International application number: PCT/US2013/066829
(87) International publication number: WO 2014/066771

(56) References cited:
- US-A- 5 312 430
- US-A- 6 024 092
- US-A- 6 024 092
- US-A1- 2009 299 327
- US-A1- 2010 094 209
- US-B2- 7 658 744
- US-B2- 7 951 111
- CHO, JH ET AL.: 'Positional Reproducibility and Effects of a Rectal Balloon in Prostate Cancer Radiotherapy' J. KOREAN MED. SCI. vol. 24, no. 5, 01 January 2009, pages 894 - 903, XP055258866 DOI: 10.3346/JKMS.2009.24.5.894
- MCGARY, JE ET AL.: 'Prostate Immobilization Using a Rectal Balloon' JOURNAL OF APPLIED CLINICAL MEDICAL PHYSICS vol. 3, no. 1, 01 January 2002, pages 6 - 11, XP055258876 DOI: 10.1120/1.1421749

## Description

### BACKGROUND

### 1. Field of the Invention

The present disclosure relates to medical apparatus. The present disclosure relates more particularly to apparatus incorporating balloons that are positioned and inflated in one cavity of the body for the purpose of stabilizing an organ located outside that cavity for treatment of the organ. While the disclosure is primarily directed to apparatus that help in the treatment of prostate and bladder cancer, the disclosure is not limited thereto.

### 2. State of the Art

Prostate cancer is a potentially lethal disease which affects nearly 200,000 men in the United States. External beam radiation therapy (EBRT) is a common method of treating prostate cancer. The goal of this treatment is to sterilize tumor cells and prevent the spread of disease beyond the prostatic capsule. For maximum efficacy, clinicians must deliver a lethal dose of radiation to the tumor while minimizing dosage to healthy surrounding tissue. This is often accomplished using fractionated treatments; i.e., a series of smaller doses delivered over a period of time. Unfortunately, the prostate, and tissue surrounding the prostate can move during and between fractionated treatments due to breathing, intestinal gas, patient movement due to discomfort or nervousness, the filling of the bladder, etc., resulting in less than maximum efficacy. As a result, it is not uncommon for a patient to be subjected to significant toxicity to normal tissue during radiation treatment. If the prostate could be immobilized, and/or if the surrounding tissue can be moved out of the treatment area, a more ideal radiation dose can be prescribed.

There are many known devices that are ostensibly adapted to aid in the positioning of the prostate for the purposes of maximizing radiation therapy. Medical balloons, such as the Foley catheter, have been expanded in the rectum in order to compress the prostate and thereby prevent prostate motion. These devices do improve treatment outcomes. At the same time, they are uncomfortable, do not necessarily permit ideal dose characteristics, and are often inconvenient for clinician usage. As a result, they have seen limited usage.

US Patent 6,024,092 discloses a catheter comprising an inflatable balloon, wherein in an inflated position said balloon includes first and second larger diameter areas defining a valley therebetween.

### SUMMARY

This summary is provided to introduce a selection of concepts that are further described below in the detailed description. This summary is not intended to identify key or essential features of the claimed subject matter, nor is it intended to be used as an aid in limiting the scope of the claimed subject matter. The invention is as defined in the appended claims.

A medical apparatus includes a catheter having an inflatable balloon at its distal end. The balloon defines at least two spaced larger diameter areas defining a valley therebetween and is adapted to be inserted in a non-inflated condition into a cavity of the human body such as the rectum, and when inflated, is adapted to cradle an organ such as the prostate located in a different body cavity. The balloon is also rotationally non-directional (symmetric) about an axis defined by the catheter so that positioning issues are limited to axial positioning rather than rotational positioning. Because of the valley in the balloon, when the balloon is inflated, only a limited portion of the rectum is necessarily pushed into contact with the prostate, and thus, the radiation toxicity to the rectum is limited. The catheter includes at least one lumen adapted for use in inflating the balloon.

In one embodiment, the catheter defines a hole distal the balloon and is provided with an additional lumen for venting fluid from distal the balloon to proximal the balloon via the hole and lumen. In one embodiment, the catheter is provided with an additional lumen adapted to receive a stiffening rod that may be used to cause the catheter to assume a desired shape (e.g., an arc) along its length.

In one embodiment, a stop element may be placed on the catheter to prevent movement of the catheter after positioning. In one embodiment where the catheter is adapted to be inserted through the rectum, the stop element is an external ring that may be moved up against the rectal opening, thereby preventing the catheter from inadvertent distal movement. In another embodiment where the catheter is adapted to be inserted through the rectum, the stop element is an internal anal stop element that is adapted to be located proximal the balloon and to prevent inadvertent proximal movement of the catheter. In one embodiment, the internal stop element is another balloon that may be inflated separately from the balloon having at least two spaced larger diameter areas defining a valley therebetween. In another embodiment, the internal stop element is a more proximal large diameter area of the balloon having at least two spaced larger diameter areas defining a valley therebetween.

In one embodiment, the balloon having at least two spaced larger diameter areas defining a valley therebetween is a single shaped plastic balloon capable of being pressurized to a pressure of at least 6 psi.

In one embodiment, the balloon having at least two spaced larger diameter areas defining a valley therebetween has the outer diameter at the larger diameter areas being between 20% and 40% larger than the outer diameter of the valley.

In one embodiment, the balloon when inflated will not substantially deform when contacting an organ through a cavity wall. In one embodiment, a balloon has a hardness of at least 80A Durometer.

In one embodiment, the balloon having at least two spaced larger diameter areas defining a valley therebetween is a single balloon having a form constricting element arranged between the ends of the balloon so that, when inflated, the balloon forms two crests about the constricting element.

In one embodiment, the balloon having at least two spaced larger diameter areas defining a valley therebetween is a single balloon forming three or more crests and valleys between adjacent crests. With multiple valleys, tissue defining the cavity in which the balloon is located has an opportunity to rest in the valleys, thus reducing radiation toxicity to that tissue.

In one aspect, bladder cancer in a woman may be treated using a catheter with a balloon as previously described by inserting the catheter into the vagina and inflating the balloon, thereby pushing the bladder away from the rectum or vice versa.

Additional aspects and advantages of the embodiments will be appreciated with reference to the detailed description taken in conjunction with the drawings.

### BRIEF DESCRIPTION OF THE DRAWINGS

Fig. 1 is a broken side view of a first embodiment of a medical apparatus.
Fig. 2 is a broken side view of the apparatus of Fig. 1 rotated by 90 degrees.
Fig. 3 is a broken cross-sectional view of the apparatus of Fig. 1 taken along section A-A of Fig. 2.
Fig. 4 is a cross sectional view of a second embodiment of a catheter for use with a medical apparatus.
Fig. 5 is a schematic of the embodiment of Fig. 4 with a shaft extending through the catheter.
Fig. 6 is a schematic of an embodiment of a first alternative balloon.
Fig. 7 is a schematic of an embodiment of a second alternative balloon with the balloon having a restrictive element extending around a mid-portion of the balloon.
Fig. 8 is a schematic of an embodiment of a third alternative balloon with the balloon having multiple crests and valleys.
Fig. 9 is a schematic of an embodiment of a medical apparatus with an external stop element.
Fig. 10 is a schematic of an embodiment of a medical apparatus with an internal stop element.

### DETAILED DESCRIPTION OF EMBODIMENTS

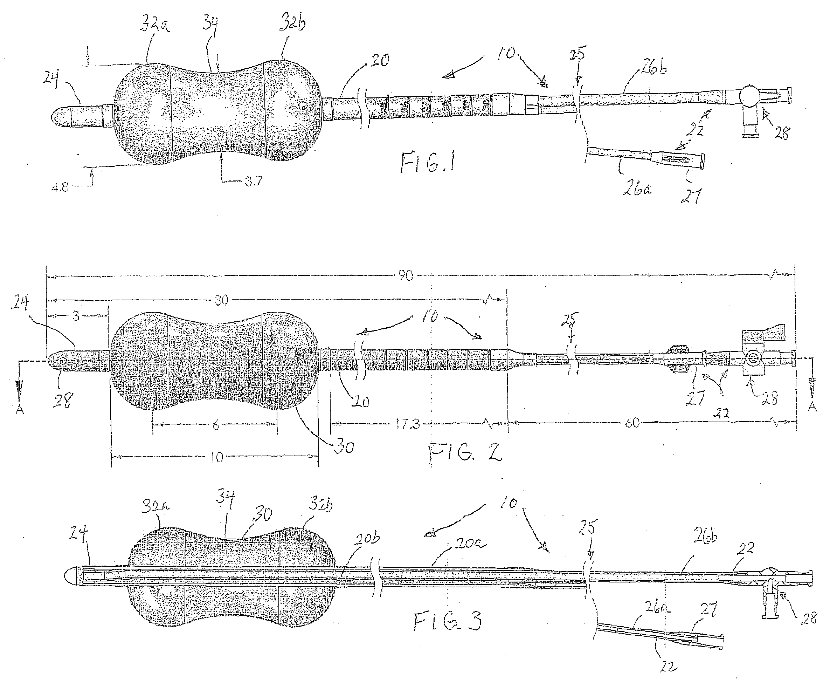

A first embodiment of a medical apparatus 10 is seen in Figs. 1-3. Medical apparatus 10 includes a multi-lumen catheter 20 with an inflation lumen 20a and a vent lumen 20b. The catheter 20 has a proximal end 22 ("proximal" being defined as closest the practitioner), a distal end 24 and an inflatable balloon 30 at the distal end 24 (at, or just proximal the distal tip 24a) that is in fluid communication with the inflation lumen 20a. At a location 25 proximal the balloon 30 but distal the proximal end 22, the multi-lumen catheter 20 breaks out into two portions or single lumen catheters 26a, 26b, with portion 26a continuing from lumen 20a and terminating at an inflation port 27, and portion 26b continuing from lumen 20b and terminating at a vent port 28. The inflation port may be a luer slip or other means that is adapted to connect to a source of fluid for the purpose of inflating the balloon 30. The vent port may be a luer slip or other means that is adapted to connect to a suction source (i.e., negative pressure source) and may be used for venting waste fluids from distal the balloon 30 to proximal the balloon. As seen best in Fig. 2, a vent hole 29 in communication with the vent port 28 may be located distal the balloon 30 at the distal end 24 of the multi-lumen catheter 20. The vent port may be coupled to a collection bag (not shown) or vented to the atmosphere. As seen in Figs. 1 and 2, the catheter 20 may be provided with markings 31 that can provide a measurement for the practitioner as to the distances from the markings to the tip (distal end 24) of the catheter 20.

In the first embodiment, the balloon 30 is generally "peanut-shaped" with two spaced larger diameter areas 32a, 32b defining a valley 34 therebetween. In one embodiment, the diameter of the larger diameter areas 32a, 32b is approximately 4.8 cm, and diameter of the valley 34 is approximately 3.7 cm, and the length of the balloon is approximately 10 cm with a peak-to-peak distance of approximately 6 cm, and the balloon is generally adapted to cradle a prostate (not shown). The distal end of the balloon 30 is located approximately 3 cm from the end of the catheter 20. As seen by comparing Figs. 1, 2, and 3, the balloon 30 is rotationally non-directional (symmetric) about a longitudinal axis of the catheter 20 so that positioning issues as discussed hereinafter are limited to axial positioning of the catheter 20 and balloon 30 rather than rotational positioning. In one embodiment, the balloon 30 is made from a plastic material having a softness of Durometer Shore 80A or higher, and is of a sufficient thickness such that it is capable of being pressurized to a pressure of at least 6 psi (41370 Pa) when it is inflated. In one embodiment, the plastic material is polyurethane, and the balloon 30 is capable of being pressurized to at least 10 psi (68950 Pa). In one embodiment, the catheter is a 1 cm outer diameter catheter.

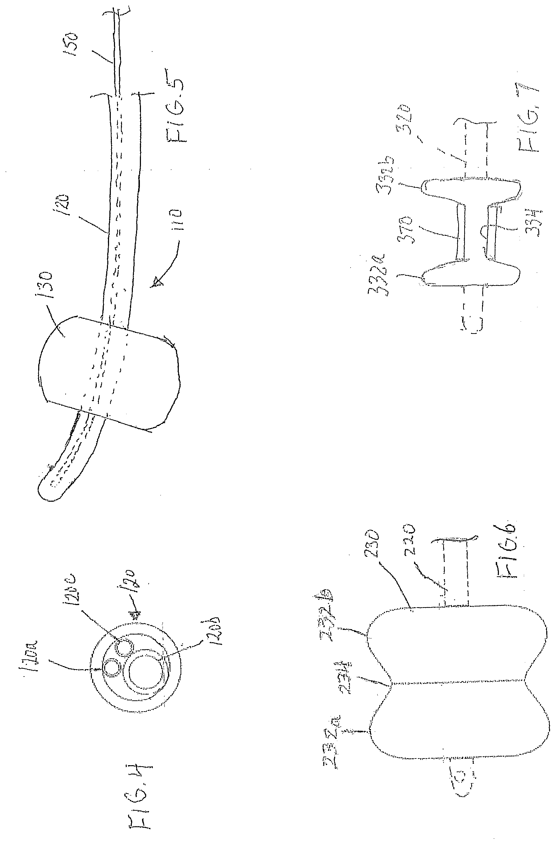

While multi-lumen catheter 20 is shown having two lumens, the catheter may have more than two lumens. A second embodiment of an apparatus 110 is seen in Figs. 4 and 5. In Fig. 4, a cross-sectional view of a second embodiment of a multi-lumen catheter 120 is seen. Catheter 120 is provided with three lumens, including an inflation lumen 120a, a vent lumen 120b, and a stiffening or introducer rod lumen 120c. Inflation lumen 120a is used to inflate a balloon 130 (Fig. 5) on the catheter shaft, and vent lumen 120b is used to vent waste fluids from distal the balloon. Introducer lumen 120c is used to receive an introducer 150 for the catheter 120 as seen in Fig. 5. In one embodiment, introducer 150 is a rod, shaft, or stylet which is optionally used to stiffen the catheter 120 for introduction into the rectum of the patient. In one embodiment, the introducer 150 is semi-rigid which for purposes of this specification and the claims means that it may be bent and shaped by the clinician but will retain its shape under normal forces experienced when introduced into the body of the patient. As suggested by Fig. 5, the introducer 150 may be bent into a shape that will be more comfortable to the patient. In one embodiment, the introducer may be formed from a shape-memory material (which for purposes herein shall also be considered "semi-rigid"). As will be discussed hereinafter, the introducer 150 may be kept in place during radiation therapy after the catheter is positioned in the rectum and removed with the catheter after a procedure, or may be removed after the catheter is properly placed in the rectum prior to application of radiation and prior to removal of the catheter from the rectum.

Turning to Fig. 6, an alternative balloon 230 is seen. Balloon 230 has two spaced larger diameter areas 232a, 232b defining a valley 234 therebetween. In one embodiment, the diameter of the larger diameter areas 232a, 232b is approximately 8 cm, and diameter of the valley 234 is approximately 6.25 cm, and the length of the balloon is approximately 7 cm with a peak-to-peak distance of approximately 5 cm. The balloon 230 is rotationally non-directional (symmetric) about a longitudinal axis of the catheter 220 (shown in phantom). In some embodiments, the balloon 230 is made from a plastic material having a softness of Durometer Shore 70A or higher, and is of a sufficient thickness such that it is capable of being pressurized to a pressure of at least 7, or 10 or 14 psi (48265, 68950 or 96530 Pa). The balloon 230 is generally adapted to cradle a prostate.

Fig. 7 is a schematic of an embodiment of a second alternative balloon 330. Balloon 330 has two spaced larger diameter areas 332a, 332b and a restrictive element 370 extending around a mid-portion of the balloon defining a valley 334 between the larger diameter areas. Restrictive element 370 may be an inelastic sleeve. In one embodiment the diameter of the larger diameter areas 332a, 332b is between 4 and 6 cm and the length of each of the larger diameter areas 332a is between 1 and 3 cm in length. In one embodiment, the outer diameter of the restrictive sleeve element is approximately 1.1cm, and the length of the restrictive sleeve element is between 4 and 6 cm. The balloon 330 is rotationally non-directional (symmetric) about a longitudinal axis of the catheter 320 (shown in phantom). In one embodiment, the balloon 330 is made from a plastic material having a softness of Durometer Shore 80A or higher, and is of a sufficient thickness such that it is capable of being pressurized to a pressure of between 14 and 17 psi (between 48265 and 96530 Pa). The balloon 330 is generally adapted to cradle a prostate.

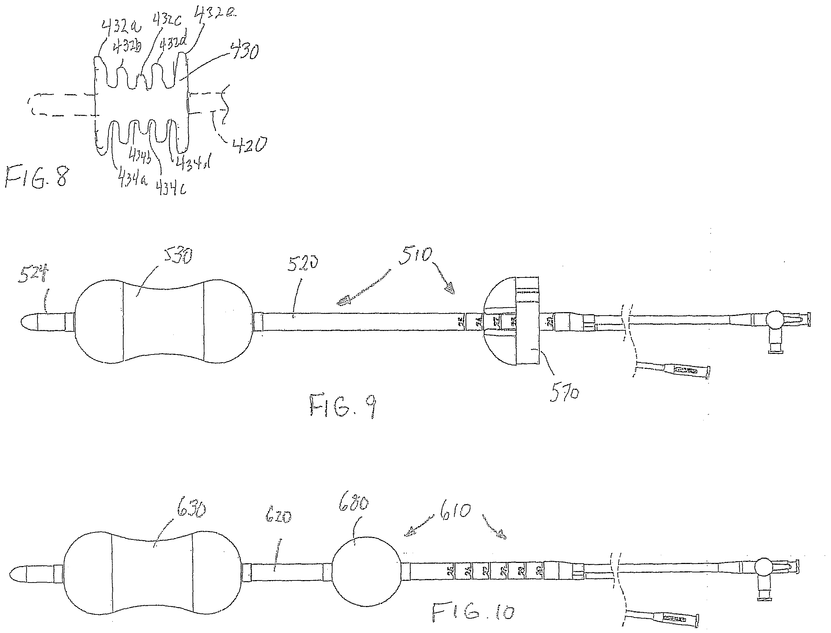

Fig. 8 is a schematic of an embodiment of a third alternative balloon 430. Balloon 430 has multiple spaced crests (larger diameter areas) 432a, 432b, 432c, 432d, 432e, and valleys 434a, 434b, 434c, 434d between the crests. In one embodiment, the most distal and most proximal crests 432a, 432e are larger in diameter than the other crests. In another embodiment, the peaks of the crests form a concave outline such that balloon 430 is generally adapted to cradle a prostate. In one embodiment, the diameter of the crests 432a, 432e is approximately 4.8 cm, the diameter of the crest 432c is approximately 3.7 cm, the diameter of valleys 434a, 434b, 434c, 434d is approximately 2 cm, and the length of the balloon is approximately 10 cm with a peak-to-peak distance from crest 432a to 432e of approximately 9 cm. The balloon 430 is rotationally non-directional (symmetric) about a longitudinal axis of the catheter 420 (shown in phantom). In one embodiment, the balloon 430 is made from a plastic material having a softness of higher than Durometer Shore 80A, and is of a sufficient thickness such that it is capable of being pressurized to a pressure of between 14 and 17 psi (between 48265 and 96530 Pa).

Turning now to Fig. 9, an apparatus 510 is seen that is similar to apparatus 10 but includes a stop element 570 that may be placed on the catheter to prevent movement of the catheter 520 after positioning. Stop element 570 may be a snap ring, an o-ring or any other large outer-diameter element that be placed over the distal end 524 of the catheter 520 and slid over the catheter and balloon 530 to a position proximal the balloon. The internal diameter of the stop element 570 is similar to the outer diameter of the catheter 510 so that it frictionally engages the catheter 510. The stop element 570 is adapted to be moved up against the rectal opening of a patient, thereby preventing the catheter from inadvertent distal movement.

An apparatus 610 similar to apparatus 10 but with an internal stop element 680 is seen in Fig. 10. Internal stop element 680 is fixed on the catheter 620 proximal the balloon 630 and is adapted to be inserted through the rectum and prevent inadvertent proximal movement of the catheter. In one embodiment, the internal stop element 680 is a second balloon that may be inflated separately from balloon 630. Where a second balloon is utilized as the internal stop element 680, the catheter 620 should be provided with a lumen for the inflation of the second balloon. In another embodiment, the internal stop element is a more proximal large diameter area of the balloon 630 which is inflated together with the balloon 630. Where the internal stop element is part of the balloon 630, if desired, a restrictive element such as sleeve 370 of Fig. 7 may be used to cause the internal stop element to be spaced from the remainder of the inflated portion of the balloon 630. In another embodiment, the internal stop element is a round or shaped plug or bead.

In one embodiment, the diameter of the spaced larger diameter areas is between substantially 4 and 10 cm, the diameter of the valley is between substantially 1 and 6 cm smaller than the diameter of the spaced larger areas, and the length of the balloon is between substantially 8 and 16 cm, wherein the term "substantially" is defined as ±10%.

In embodiments, the diameters of the spaced larger diameter areas of the balloon are different. In one embodiment, the more distal larger diameter balloon area has a larger diameter than the more proximal larger diameter balloon area. In one aspect a larger diameter distal balloon area can push distal seminal vesicles further from a radiation site, while a relatively smaller larger diameter proximal balloon area can reduce the urination urge of a patient. In some embodiments, both larger diameter balloon areas are between 20% and 40% larger in outer diameter than the outer diameter of the valley defined between the larger diameter areas.

In one aspect, aspects of the described embodiments may be used in conjunction with each other. Thus, by way of example only and not by way of limitation, different embodiments of the balloons 30, 130, 230, 330, 430, 530, 630 may be used in conjunction with different catheters. Similarly, markings on catheter 20 can be used in conjunction with any of catheters 120, 220, 320, 420, 520, 620. Internal and/or external stop elements 570, 680 may be used in conjunction with any of the described balloons and any of the catheters.

In one embodiment, apparatus 10, 110, 510, 610 using any of balloons 30, 130, 230, 330, 430, 530, 630 is used as follows. With respect to EBRT, before radiation treatment begins a planning or simulation session is required to pinpoint the tumor and determine the treatment sequence. A support mold is created that supports the back, pelvis and thighs of the patient to ensure accurate positioning over the course of radiation treatments. A tomographic (CT) scan of the pelvis is taken while the patient is supported by the mold and the CT scan is used to generated a 3D image of the pelvic anatomy including the prostate, bladder, rectum and pelvic bones. An x-ray simulator is then used to provide a picture of the tumor site and help determine how the radiation will be directed to it. The beams of the simulator are positioned to deliver the appropriate dose of radiation to the prostate while sparing surrounding healthy tissues and structures. Beam positioning is then verified using fluoroscopy. Using the x-rays as a guide, a radiation therapist may make marks on the treatment area on the patient's skin that are to be used as a guide during treatment. Treatment is accomplished over a period of weeks; usually five days a week for six to eight weeks. Each procedure during the treatment period lasts minutes at a time. The procedure involves positioning the patient in the mold on an x-ray table according to the map on the patient's skin and the information obtained from the simulation. Where a linear accelerator is used for treatment, the linear accelerator is activated and moves in a circular fashion around the tumor area of the patient. The radiation is applied over a period of minutes.

The apparatus 10, 110, 510, 610 using any of balloons 30, 130, 230, 330, 430, 530, 630 may be used during treatment, during x-ray simulation, and during CT scanning, if desired. In each situation, an introducer or shaft 150 is optionally shaped as desired, and optionally inserted into a lumen of the apparatus 10, 110, 510, 610 (e.g., into vent port lumen 20b via catheter portion 26b and vent port 29, or into introducer lumen 120c). The apparatus with the optionally inserted shaft 150 (and with the balloon in a deflated condition) is inserted through the rectal opening of the patient and moved to a location adjacent the prostate. In the case of use during treatment, the distance (extent of movement) of the apparatus into the rectum may have been predetermined either by previous experience during x-ray simulation and/or CT scanning, or by having taken measurements from the CT scan, and the markings (e.g., markings 31) may be used to establish positioning. Once the catheter and balloon are properly positioned axially, the introducer or shaft 150, if utilized, may be removed. Because the balloon is symmetric about the longitudinal axis of the catheter, there is no need to rotationally position the catheter and balloon. The balloon may then be inflated by injecting air or other fluid through the inflation port of the apparatus such that the balloon inflates and cradles the prostate, even though the rectum wall is located between the balloon and the prostate. The cradling of the prostate advantageously prevents movement of the prostate during a procedure. Also, because the balloon is provided with one or more valleys when the balloon is inflated, only a limited portion of the rectum is necessarily pushed into contact with the prostate, and thus, radiation toxicity to the rectum is limited during application of radiation. Prior to or after inflation of the balloon, an external stop 570, if available, may be moved into position against the outside of the rectal opening to prevent inadvertent distal movement of the apparatus. In addition, prior to, during, or after inflation of the balloon, an internal stop 580, if available, may be inflated inside the rectum to prevent inadvertent proximal movement of the apparatus. While the balloon is inflated, if desired, in some embodiments, negative pressure may be applied at the vent port so that digestive waste in the rectum that is distal the balloon may pass into the vent hole, through the catheter and out the vent port. After radiation is completed, the balloon (and internal stop 580, if any) is deflated via the inflation port, and the catheter may be removed from the patient by pulling on it proximally.

In one aspect, bladder cancer in a woman may be treated using a catheter with an apparatus as previously described by inserting the catheter of the apparatus into the vagina and inflating the balloon, thereby pushing the bladder away from the rectum or vice versa.

In one aspect, one or more diagnostic elements may be provided on the balloon of the apparatus. In one embodiment, one or more dosimeters are placed on the surface of the balloon. Where multiple dosimeters are utilized, the dosimeters may be arranged in an array or otherwise. The dosimeters may be spaced axially and circumferentially and may be located on the inside or outside surface of the balloon. In one embodiment the dosimeters are film dosimeters with films that increase in optical density in response to incident radiation. In another embodiment the one or more diagnostic elements include one or more pressure sensors. In one embodiment, multiple diagnostic elements of different types are utilized together. By way of example only, one or more dosimeters and one or more pressure sensors may be placed on the balloon of the apparatus.

In another aspect, one or more therapeutic elements may be provided on the balloon of the apparatus. In one embodiment, the balloon of the apparatus is provided with an agent such as a drug that may be beneficial to the rectal mucosa. By way of example only, the drug may be a poly (ADP-ribose) polymerase. The drug may be placed on the outer wall of the balloon and may elute when the balloon is pushed with force against the wall of the colon. Also by way of example only, the drug may be an oxygenation-decreasing drug that protects the healthy rectal mucosa.

There have been described and illustrated herein several embodiments of an apparatus and a method for treatment of an organ. While particular embodiments have been described, it is not intended that the teachings be limited thereto, as it is intended that the invention be as broad in scope as the art will allow and that the specification be read likewise. Thus, while particular embodiments of balloons with particular sizes and materials have been disclosed, it will be appreciated that other embodiments of balloons with other sizes and materials may be used as well. Also, while single balloons that are shaped to cradle the prostate have been disclosed, it will be appreciated that the single balloons can be replaced with multiple balloons that are arranged to simulate the single balloons. The multiple balloons can be individually inflatable and deflatable. It will therefore be appreciated by those skilled in the art that yet other modifications could be made to the provided without deviating from its scope of the claims.

## Claims

1. A medical apparatus (10,110,510,610), comprising:
a catheter (20,120,220,320,420,520,620) defining a first lumen (20a,120a) and having a proximal end (22), a distal end (24), and a longitudinal axis (A); and
at least one inflatable balloon (30,130,230, 430,530,630) located about said distal end, said at least one inflatable balloon being in communication with said first lumen and having a deflated position and an inflated position, wherein in said inflated position said at least one balloon includes first and second larger diameter areas (32a,32b,232a,232b,332a,332b,432a,432e) defining a valley (34,234,334,434a,434d) therebetween, said at least one inflatable balloon being symmetrical about said longitudinal axis (A), said at least one inflatable balloon adapted to being pressurized to a pressure of at least 41370Pa (6 psi), the length of said at least one inflatable balloon being between 8 cm and 16 cm and the outer diameters of said first and second spaced larger diameter areas being between 4 and 10 cm and between 20% and 40% larger than an outer diameter of said valley.

2. A medical apparatus (10,110,510,610) according to claim 1, wherein: said at least one inflatable balloon (30,130,230, 430,530,630) comprises a single balloon having said first and second spaced larger diameter areas (32a,32b,232a,232b, 332a,332b,432a-e) defining a valley (34,234,334,434a-d) therebetween.

3. A medical apparatus (10) according to claim 2, wherein: said at least one inflatable balloon (30) is substantially 10 cm long, said outer diameter of said first and second spaced larger diameter areas (32a,32b) is substantially 4.8 cm, and a distance between locations of greatest diameter of said two spaced larger diameter areas (32a,32b) is substantially 6 cm.

4. A medical apparatus (10) according to claim 3, wherein: said valley (34) has an outer diameter of substantially 3.7 cm.

5. A medical apparatus (10,510,610) according to claim 2, wherein: said single balloon (30,530,630) is substantially "peanut-shaped".

6. A medical apparatus according to claim 1, wherein: said at least one inflatable balloon comprises a single balloon, and said medical apparatus further comprises a sleeve (370) fitted over a center area of said balloon, said sleeve preventing said balloon from expanding at said center area and thereby defining said valley (334).

7. A medical apparatus according to claim 1, wherein: said at least one inflatable balloon (430) includes at least third and fourth spaced larger diameter areas (432b,432c,432d) defining additional valleys (434b,434c), said third and fourth spaced larger diameter areas (432b,432c,432d) having diameters smaller than said diameter of said first and second spaced larger diameter areas (432a,432e).

8. A medical apparatus (10,110,510,610) according to any previous claim, wherein: said catheter (20) defines a second lumen (20b,120b) and a hole distal said at least one inflatable balloon, said hole being in fluid communication with said second lumen.

9. A medical apparatus (10,110,510,610) according to any previous claim, further comprising: an introducer (150) adapted to extend into said catheter.

10. A medical apparatus (10,110,510,610) according to claim 9, wherein: said introducer (150) is semi-rigid.

11. A medical apparatus (10,110,510,610) according to claim 8, wherein: said catheter (120) defines a third lumen (120c), and said medical apparatus further comprises a semi-rigid introducer (150) adapted to extend into said third lumen.

12. A medical apparatus (510,610) according to any previous claim, further comprising at least one of (i) an outer stop element (570) movable relative to said longitudinal axis and extending about said catheter in frictional engagement with said catheter, said stop element adapted to engage an external body wall and prevent distal movement of said catheter, and (ii) an inner stop element (680) proximal of said balloon and adapted to engage an internal body wall and prevent proximal movement of said catheter when inflated and engaged.

13. A medical apparatus (10,110,510,610) according to any previous claim, wherein: said catheter (10,510,610) includes distance-to-tip spaced markings proximal of said balloon.

14. A medical apparatus (10,110,510,610) according to any previous claim, wherein: said balloon (30,130,230, 430,530,630) is a plastic balloon having a hardness of at least Durometer Shore 80A.

15. A medical apparatus according to any previous claim wherein: said outer diameter of a distal one of said first and second larger diameter areas is larger than said outer diameter of a proximal one of said first and second larger diameter areas.

## Patentansprüche

1. Medizinische Vorrichtung (10,110,510,610), umfassend:
einen Katheter (20,120,220,320,420,520,620), der ein erstes Lumen (20a, 120a) definiert und ein proximales Ende (22), ein distales Ende (24) und eine Längsachse (A) aufweist; und
mindestens einen aufblasbaren Ballon (30,130,230, 430,530,630), der sich um das distale Ende befindet, wobei der mindestens eine aufblasbare Ballon in Kommunikation mit dem ersten Lumen ist und eine entleerte Position und eine aufgeblasene Position aufweist, wobei in der aufgeblasenen Position der mindestens eine Ballon erste und zweite Gebiete (32a,32b,232a,232b,332a,332b,432a,432e) größeren Durchmessers enthält, die ein Tal (34,234,334,434a,434d) dazwischen definieren, wobei der mindestens eine aufblasbare Ballon symmetrisch um die Längsachse (A) ist, wobei der mindestens eine aufblasbare Ballon angepasst ist, um mit einem Druck von mindestens 41370Pa (6 psi) beaufschlagt zu werden, wobei die Länge des mindestens einen aufblasbaren Ballons zwischen 8 cm und 16 cm liegt und die äußeren Durchmesser der ersten und zweiten beabstandeten Gebiete größeren Durchmessers zwischen 4 und 10 cm und zwischen 20 % und 40 % größer als ein äußerer Durchmesser des Tals sind.

2. Medizinische Vorrichtung (10,110,510,610) nach Anspruch 1, wobei: der mindestens eine aufblasbare Ballon (30,130,230, 430,530,630) einen einzelnen Ballon umfasst, der die ersten und zweiten beabstandeten Gebiete (32a,32b,232a,232b, 332a,332b,432a-e) größeren Durchmessers, die ein Tal (34,234,334,434a-d) dazwischen definieren, aufweist.

3. Medizinische Vorrichtung (10) nach Anspruch 2, wobei: der mindestens eine aufblasbare Ballon (30) im Wesentlichen 10 cm lang ist, der äußere Durchmesser der ersten und zweiten beabstandeten Gebiete (32a,32b) größeren Durchmessers im Wesentlichen 4,8 cm ist, und ein Abstand zwischen Orten größten Durchmessers der zwei beabstandeten Gebiete (32a,32b) größeren Durchmessers im Wesentlichen 6 cm ist.

4. Medizinische Vorrichtung (10) nach Anspruch 3, wobei: das Tal (34) einen äußeren Durchmesser von im Wesentlichen 3,7 cm aufweist.

5. Medizinische Vorrichtung (10,510,610) nach Anspruch 2, wobei: der einzelne Ballon (30,530,630) im Wesentlichen "Erdnussform" aufweist.

6. Medizinische Vorrichtung nach Anspruch 1, wobei: der mindestens eine aufblasbare Ballon einen einzelnen Ballon umfasst, und die medizinische Vorrichtung weiter eine Hülse (370) umfasst, eingepasst über ein mittiges Gebiet des Ballons, wobei die Hülse den Ballon am Expandieren beim mittigen Gebiet hindert und wodurch das Tal (334) definiert wird.

7. Medizinische Vorrichtung nach Anspruch 1, wobei: der mindestens eine aufblasbare Ballon (430) mindestens dritte und vierte beabstandete Gebiete (432b,432c,432d) größeren Durchmessers enthält, die zusätzliche Täler (434b,434c) definieren, wobei die dritten und vierten beabstandeten Gebiete (432b,432c,432d) größeren Durchmessers Durchmesser aufweisen, die kleiner als die Durchmesser der ersten und zweiten beabstandeten Gebiete (432a,432e) größeren Durchmessers sind.

8. Medizinische Vorrichtung (10,110,510,610) nach einem vorstehenden Anspruch, wobei:
der Katheter (20) ein zweites Lumen (20b, 120b) und eine Öffnung definiert, distal zu dem mindestens einen aufblasbaren Ballon, wobei die Öffnung in fluider Kommunikation mit dem zweiten Lumen ist.

9. Medizinische Vorrichtung (10,110,510,610) nach einem vorstehenden Anspruch, weiter umfassend: eine Einführvorrichtung (150), die angepasst ist, um sich in den Katheter zu erstrecken.

10. Medizinische Vorrichtung (10,110,510,610) nach Anspruch 9, wobei: die Einführvorrichtung (150) halbstarr ist.

11. Medizinische Vorrichtung (10,110,510,610) nach Anspruch 8, wobei: der Katheter (120) ein drittes Lumen (120c) definiert, und die medizinische Vorrichtung weiter eine halbstarre Einführvorrichtung (150) umfasst, die angepasst ist, um sich in das dritte Lumen zu erstrecken.

12. Medizinische Vorrichtung (510,610) nach einem der vorstehenden Anspruch, weiter umfassend mindestens eines von (i) einem äußeren Stoppelement (570), das zu der Längsachse relativ beweglich ist und sich um den Katheter in Reibungseingriff mit dem Katheter erstreckt, wobei das Stoppelement zum Eingriff mit einer äußeren Körperwand und zum Hindern einer distalen Bewegung des Katheters angepasst ist, und (ii) einem inneren Stoppelement (680), das von dem Ballon proximal ist und zum Eingriff mit einer inneren Körperwand angepasst ist und eine proximale Bewegung des Katheters hindert, wenn aufgeblasen und im Eingriff.

13. Medizinische Vorrichtung (10,110,510,610) nach einem der vorstehenden Anspruch, wobei: der Katheter (10,510,610) proximal von dem Ballon Abstand-zur-Spitze beabstandete Markierungen enthält.

14. Medizinische Vorrichtung (10,110,510,610) nach einem der vorstehenden Anspruch, wobei:
der Ballon (30,130,230, 430,530,630) ein Kunststoff-Ballon ist, der eine Härte von mindestens Durometer Shore 80A aufweist.

15. Medizinische Vorrichtung nach einem der vorstehenden Anspruch, wobei: der äußere Durchmesser eines distalen von den ersten und zweiten Gebieten größeren Durchmessers größer ist, als der äußere Durchmesser eines proximalen von den ersten und zweiten Gebieten größeren Durchmessers.

## Revendications

1. Appareil médical (10,110,510,610), comprenant :
un cathéter (20,120,220,320,420,520,620) définissant une première lumière (20a,120a) ayant une extrémité proximale (22), une extrémité distale (24), et un axe longitudinal (A) ; et
au moins un ballonnet gonflable (30,130,230,430,530,630) situé autour de ladite extrémité distale, ledit au moins un ballonnet gonflable étant en communication avec ladite première lumière et ayant une position dégonflée et une position gonflée, dans lequel dans ladite position gonflée ledit au moins un ballonnet comprend des première et deuxième zones de plus grand diamètre (32a,32b,232a,232b,332a,332b,432a,432e) définissant un creux (34,234,334,434a,434d) entre elles, ledit au moins un ballonnet gonflable étant symétrique autour dudit axe longitudinal (A), ledit au moins un ballonnet gonflable étant adapté pour être mis sous pression à une pression d'au moins 41 370 Pa (6 psi), la longueur dudit au moins un ballonnet gonflable étant comprise entre 8 cm et 16 cm et les diamètres extérieurs desdites première et deuxième zones espacées de plus grand diamètre étant compris entre 4 et 10 cm et entre 20 % et 40 % plus grands qu'un diamètre extérieur dudit creux.

2. Appareil médical (10,110,510,610) selon la revendication 1, dans lequel : ledit au moins un ballonnet gonflable (30,130,230,430,530,630) comprend un ballonnet unique ayant lesdites première et deuxième zones espacées de plus grand diamètre (32a,32b,232a,232b,332a,332b,432a-e) définissant un creux (34,234,334,434a-d) entre elles.

3. Appareil médical (10) selon la revendication 2, dans lequel : ledit au moins un ballonnet gonflable (30) est sensiblement d'une longueur de 10 cm, ledit diamètre extérieur desdites première et deuxième zones espacées de plus grand diamètre (32a,32b) est sensiblement de 4,8 cm, et une distance entre des emplacements de plus grand diamètre desdites deux zones espacées de plus grand diamètre (32a,32b) est sensiblement de 6 cm.

4. Appareil médical (10) selon la revendication 3, dans lequel : ledit creux (34) a un diamètre extérieur de sensiblement 3,7 cm.

5. Appareil médical (10,510,610) selon la revendication 2, dans lequel : ledit ballonnet unique (30,530,630) est sensiblement « en forme d'arachide ».

6. Appareil médical selon la revendication 1, dans lequel : ledit au moins un ballonnet gonflable comprend un ballonnet unique, et ledit appareil médical comprend en outre un manchon (370) ajusté sur une zone centrale dudit ballonnet, ledit manchon empêchant le déploiement dudit ballonnet au niveau de ladite zone centrale et définissant ainsi ledit creux (334).

7. Appareil médical selon la revendication 1, dans lequel : ledit au moins un ballonnet gonflable (430) comprend au moins des troisième et quatrième zones espacées de diamètre plus grand (432b,432c,432d) définissant des creux supplémentaires (434b,434c), lesdites troisième et quatrième zones espacées de plus grand diamètre (432b,432c,432d) ayant des diamètres plus petits que ledit diamètre desdites première et deuxième zones espacées de plus grand diamètre (432a,432e).

8. Appareil médical (10,110,510,610) selon l'une quelconque des revendications précédentes, dans lequel : ledit cathéter (20) définit une deuxième lumière (20b,120b) et un trou distal dudit au moins un ballonnet gonflable, ledit trou étant en communication fluidique avec ladite deuxième lumière.

9. Appareil médical (10,110,510,610) selon l'une quelconque des revendications précédentes, comprenant en outre : un dispositif d'introduction (150) adapté pour se déployer dans ledit cathéter.

10. Appareil médical (10,110,510,610) selon la revendication 9, dans lequel : ledit dispositif d'introduction (150) est semi-rigide.

11. Appareil médical (10,110,510,610) selon la revendication 8, dans lequel : ledit cathéter (120) définit une troisième lumière (120c), et ledit appareil médical comprend en outre un dispositif d'introduction semi-rigide (150) adapté pour s'étendre dans ladite troisième lumière.

12. Appareil médical (510,610) selon l'une quelconque des revendications précédentes, comprenant en outre au moins l'un de (i) un élément d'arrêt extérieur (570) mobile par rapport au dit axe longitudinal et s'étendant autour dudit cathéter en prise par frottement avec ledit cathéter, ledit élément d'arrêt étant adapté pour se mettre en prise avec une paroi de corps extérieure et empêcher le mouvement distal dudit cathéter, et (ii) un élément d'arrêt intérieur (680) proximal dudit ballonnet et adapté pour se mettre en prise avec une paroi de corps intérieure et empêcher le mouvement proximal dudit cathéter lorsqu'il est gonflé et en prise.

13. Appareil médical (10,110,510,610) selon l'une quelconque des revendications précédentes, dans lequel : ledit cathéter (10,510,610) comprend des repères espacés d'une distance par rapport à la pointe proximaux dudit ballonnet.

14. Appareil médical (10,110,510,610) selon l'une quelconque des revendications précédentes, dans lequel : ledit ballonnet (30,130,230, 430,530,630) est un ballonnet en plastique ayant une dureté au duromètre Shore A d'au moins 80.

15. Appareil médical selon l'une quelconque des revendications précédentes, dans lequel : ledit diamètre extérieur de l'une distale desdites première et deuxième zones de plus grand diamètre est plus grand que ledit diamètre extérieur de l'une proximale desdites première et deuxième zones de plus grand diamètre.
